# EUROPEAN PATENT SPECIFICATION

(11) **EP 1 661 565 B1**
(45) Date of publication and mention of the grant of the patent: **24.10.2007**
(21) Application number: 04762296.4
(22) Date of filing: 03.08.2004
(51) Int. Cl.: A61K 31/401, A61P 17/00

(54) **COMPOSITION COMPRISING L-PROLINE FOR THE TREATMENT OF VITILIGO**
ZUSAMMENSETZUNG ENTHALTEND L-PROLINE ZUR BEHANDLUNG VON VITILIGO& x9;& x9;
COMPOSITION CONTENANT L-PROLINE POUR LE TRAITEMENT DU VITILIGO

(30) Priority: 05.08.2003 CU 17703
(43) Date of publication of application: 31.05.2006
(73) Proprietor: Centro de Histoterapia Placentaria, 11300 Ciudad de la Habana (CU)
(72) Inventor: MIYARES CAO, Carlos Manuel;, 11300 Ciudad de la Habana; (CU); HOLLANDS BARCA, Ileana;, 11300 Ciudad de la Habana; (CU); PIMIENTA LOPEZ, Reina, 3ra e/ 178 y 180 Edif. B4, 12100 Ciudad de la Habana (CU)
(74) Representative: van Loon, C.J.J.
(86) International application number: PCT/CU2004/000008
(87) International publication number: WO 2005/011676

(56) References cited:
- EP-A1- 1 342 474
- WO-A-00/15202
- WO-A1-00/06180
- WO-A2-20/04026295

## Description

### Field of invention

The present invention is related with the field of dermatology and in particular with a composition that may be used in area of cosmetics as well as in that of human medicine. It is effective in lesions caused by vitiligo.

### Antecedents of the invention

Melanogenesis is a process by which the reproduction of cells producers of dermal pigment (melanocites) and the amount of granules of the melanic pigment that such cells synthesize, increase.

There are diseases such as vitiligo (Prcic S. Duran V. Et Al, 2002), Panuncio Al. et al, 2003), Gauthier Y. Et al, 2003) in which a progressive loss of skin melanic pigment producing cells takes place, after the subject has been under severe stress (Martin-Garcia RF. Et al, 2003), (Hadad Al. Et al, 2003), (Briganti S.et al, 2003).

The disease affects approximately 1% of world population and it is characterized by the appearance of white patches, mainly located on the face and limbs, disregarding age, sex or geographic region.

From the state of the art it is known that certain chemical substances, which stimulate the melanogenesis process, are used for the treatment of this disease. Among them are those called psoralenes (Kreuter A. Et al, 2001), (Handa S. Et al, 2001), (Hofer A. Et al, 200), (K wok YK. et al, 2002), synthetic derivatives from an Egyptian plant (Ammi-Majus).

However psoralenes are not exempt from toxic side effects, being capable of causing dermatitis and necrosis of the skin after topic application. Besides, it has a slow reversible effect after discontinuing medication in most of the cases.

Another technical solution analogous to the present invention is that referred in Patent No. FR 8220746. In this patent the obtainment of a 50% alcoholic extract from human placenta, collected from healthy pregnant women after delivery, at aseptic conditions.

From the state of the art is also known; Melagenina Plus®, Patent No. WO 00/06180 "Composition for the stimulation of the synthesis of skin melanic pigment, obtainment procedure and use", that it is a 50% alcoholic extract with calcium chloride, from human placenta, which has been the most effective and innocuous of all treatments so far. (Miyares Cao. Carlos, 1997).

The treatment with Melagenina plus is topic, once a day and without sunlight exposure. Its active principle is an alpha lipoprotein obtained from human placental cotyledons, by processing them with organic dissolvent and later redisolution in an ethanol and calcium chloride solution.

L-Proline is not a common aminoacid because its structure is considered as an iminoacid, since its NH group is compromised with adjacent carbons. Hence it is considered as a α-amino acid with 115.1 molecular weight.

So far there is no report in the state of the art that vincule the use of Proline with the increase of the production of melanocytes or to bring about provoque skin repigmentation in patients suffering from vitiligo.

### Description of the invention

L- Proline is the active principle of the subject (objeto) composition of the present invention, which stimulates the synthesis of skin melanic pigment and the reproduction of melanocytes, without serious side effects as demonstrated by toxicological and clinical trials performed.

The present invention has surprisingly demonstrated that that L - Proline has an effect qualitatively superior in the process of melanogenesis, compared with the products already existing in the state of the art above mentioned. This product stimulates the synthesis of skin melanic pigment and the reproduction of melanocytes as demonstrated by pharmacologic assays. No severe adverse reactions were reported as demonstrated by toxicological, teratologic and clinical trials. Easy to obtain and application, the repigmentant effect of this product starts rapidly (15 to 20 days of treatment) and it is irreversible once the application of the product is discontinued. After repigmentation, color of the skin treated is identical to that of the normal skin areas, which do not increase the intensity of its coloring with the time.

According to clinical trials performed with the composition of the invention, Proline has demonstrated encouraging results for the treatment of vitiligo and its use has shown remarkable notable effectiveness in all patients treated. This product is administered topically just 3 times a week, something that had never been achieved before, in the treatment of this disease.

The process for the obtainment of the subject of invention composition was performed by using an L-Proline (Sigma) solution, reagent quality, in a 0. 5-2-mg/ml - concentration range. For this, 70°-90° ethanol was used and it was kept at 22- 28° C in 120 ml amber glass bottles.

### EXAMPLE 1

Melanogenic activity of the 80% hydro alcoholic solution and 1.5 mg/ml L-Proline was determined, compared with Melagenina Plus on melanocytes of the epidermis basal layer of the ears of male black mice of 18-22 g of the C57BL6 strain supplied by CENPALAB.

Each product trial was repeated was repeated three times.

The histochemical analisis in the epidermis of the ears of such mice evidenced the the changes in the countings of melanocytes present in such tissue (Nordlund et al, 1981).

Grups of 5 animals each were formed to perform these trials. The samples trailed were:
A- control-80° Alcohol
B- Mela genina plus
C- L -Proline hydroalcoholic solution

The study was performed by the topic application on mice

The average weight of the animals which were kept for acclimatising at the Bioterio for 10 days, was 20.15 g. Omce the 5 applications were concluded, 72 hours after the last one the animals were killed (sacrificed) by traction of cervical vertebrae.

After sacrifice, both ears were depilled with a depillating cream which is removed 10 minutes after application and then ears are washed with abundant tap water. The first step to obtain the epidermis of each ear and submit it to the incubation histochemical method in L- Dopa, consists of removing the skin from the cartilage, manually, then skin of each ear is introduce into a Calcium Cloride 1M solution at room temperature for 1-2 hours, which facilitates dermis-epidermis separation. Once this is achieved, the epidermis is washed with distilled water for 10 minutes and the process is started:

The epidermis is sunk in 85° alcohol for 20 minutes.

It is washed with distilled water

It is submitted to an L-Dopa solution (1mg/mL)dissolved in buffer phosphate at 37° C.

This solution is renewed and the epidermis remains in there for 12-15 hours. Then it is washed with abundant tap water remove the excess of Dopa from the tissue.

It is washed with disilled water once again

It is fixed in 10% Neutral Formol for 20 minutes

Again washed in distilled water

At the end it is montada in a simipermanente montage solution

Five different areas of the ear were chosen to determine the number of melanocytes/mm². The areas with larger number of melanocytes were chosen and the part of the DIGIPAT program, specific for cell counting, was used. The quantity of melanocytes/mm² in each of them was determined, so that 10 values were obtained, and hence 50 for each group.

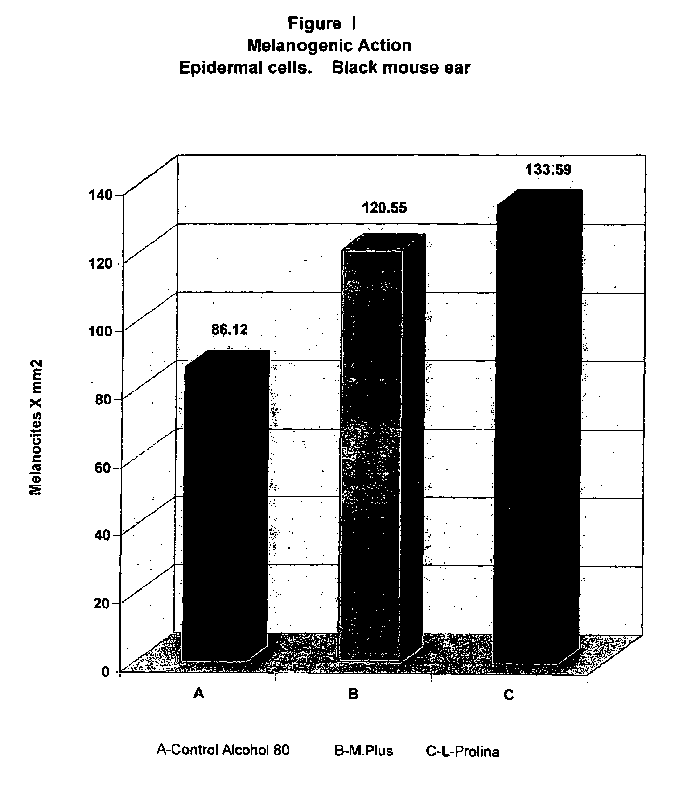

Assays were performed to determine L-Proline melanogenic action, compared with Melagenina Plus. As shown in fig. 1, it has been demonstrated that owns a marked notable superior melanogenic effect on the melanocytes of the ear of black mice over Melagenina Plus.

Results from experiments show that the amino acid L-Proline stimulates the reproduction of melanocytes and the synthesis of the skin melanic pigment when applied topically in hydroalcoholic solution. Thus a quantitatively higher value is obtained, compared with Melagenina Plus and the alcohol (80°).

### EXAMPLE 2

The number of 21 patients suffering from vitiligo were chosen for the clinical trial in humans, under the principle of wilfulness. All of them were from the servicio clinico nacional del centro and were distributed as follows:
Sex
   Female: 10
   Male: 11
Race
   White: 16
   Black: 4
   Half breed: 1
Age
   Younger the 15: 4
   Older than 15: 17
Percent of depigmentation:
   Below 10%: 14
   Between 10 and 20%: 3
   Between 20 and 40%: 4

It is important to note that all patients chosen had been Melagenina Plus treatment-resistant for a minimum at list for one year.

The treatment consisted of the application of an L-proline hydroalcoholic solution at 80%, at a 1.5 mg/ml concentration, by rubbing with the fingers on the skin depigmented area, 3 times a week (Mondays Wednesdays and Fridays).

The innocuousness of the product to be used in the trial was demonstrated by appropriate corresponding dermal and ophthalmic irritability.

A clinical record was prepared for each patient at the beginning of the experiment and digital pictures of the depigmentd area were taken, where the application of L-Proline hydroalcoholic solution was indicated.

One month later the patients were called to check the treatment effects. This was repeated at 3 and 6 months of treatment with the investigational product.

The repigmentant effect obtained was determined in each appointment and it was evaluated as follows:
Notable:
   Decrease of the % of body depigmentation
   Appearance of freckle-like pigment within or at the edges of the lesion
   Decrease of the lesion size
Moderate:
   Opacification of the color of the lesion without disappearing, or decreasing
Mala (Poor)
   No change

Finally digital pictures were taken again in the treated area of cases in which outstanding apreciables changes in repigmentation took place.

This was a 6-month clinical trial.

Outcomes of the clinical trial

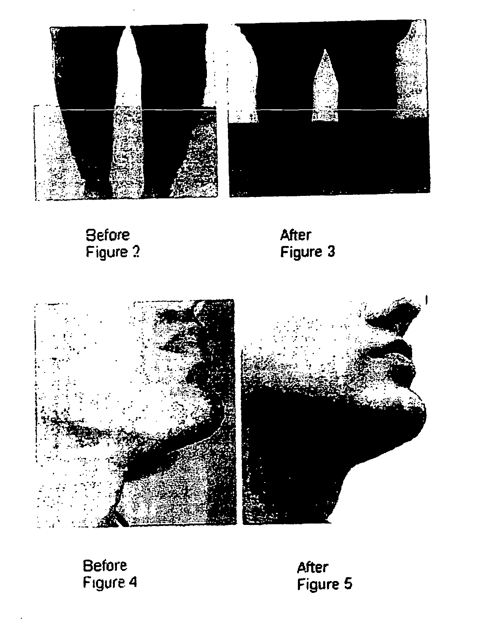

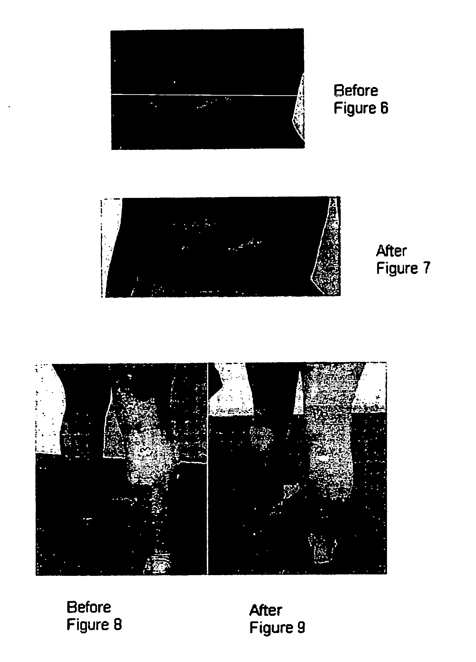

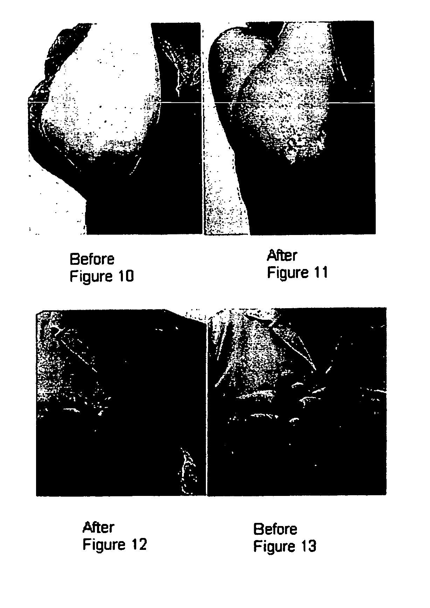

The table below shows the results obtained as well as well as the pictures (Figures 2-13).

**Table # 1.Outcomes of repigmentation**

| No. | Total Repigmentation | Partial Repigmentation | Without Repigmentation | Increase Of lesions | Effect Evaluation |
|---|---|---|---|---|---|
| 1.- | - | X | - | - | Noteworthy |
| 2.- | - | X | - | - | Noteworthy |
| 3.- | - | X | - | - | Noteworthy |
| 4.- | - | X | - | - | Noteworthy |
| 5.- | - | X | - | - | Noteworthy |
| 6.- | - | X | - | - | Noteworthy |
| 7.- | - | X | - | - | Noteworthy |
| 8.- | - | X | - | - | Noteworthy |
| 9.- | - | X | - | - | Noteworthy |
| 10.- | - | X | - | - | Noteworthy |
| 11.- | - | X | - | - | Moderate |
| 12.- | - | X | - | - | Moderate |
| 13.- | - | X | - | - | Moderate |
| 14.- | - | X | - | - | Moderate |
| 15.- | - | X | - | - | Moderate |
| 16.- | - | X | - | - | Moderate |
| 17.- | - | X | - | - | Moderate |
| 18.- | - | X | - | - | Moderate |
| 19.- | - | X | - | - | Poor |
| 20.- | Drop out | X | - | - | Poor |
| 21.- | | X | - | - | |

As shown in the table above, Just 2 patients of 22 did not show response to the treatment and 18 of them showed noteworthy to moderate response Just one patient dropped out so that his results could not be evaluated. On the other hand, it should be noted that no increase of lesions or local or systemic side effect were reported in any of the cases. The results obtained backed up (demonstrated) therapeutic effectiveness as well as innocuousness of L-proline in hydroalcoholic solution. The fact that most of the patients showed positive response by using it just 3 times a week shows its superiority, compared with Melagenina Plus, even more if it is considered that all of them had been refractory to such product when used daily.

Another advantage of this compound over Melagenina Plus is its synthetic origin which avoids difficult and costly tests usually performed to placentas used in Melagenina Plus obtainment process, to prevent contaminations.

### Example 3

Spectrophtometric determinations and of aminoacids analysis of L-Proline hydroalcoholic solution, just prepared (fresh) and at 12 and 24 days after elaboration, were performed to carry out stability studies. According to previous techniques it was demonstrated that L-Proline remains stable for 2 years after preparing the solution. (Tables 2, 3).

**Table # 2Results of the spectrophotometric stability study (por fotometria)**

| ***product*** | ***Spectrum determination*** | | | |
|---|---|---|---|---|
| | ***(nm)*** | ***Fresh*** | ***12 months*** | ***24 months*** |
| L-Proline | 200-205 | 0.051 UA | 0.072UA | 0.071 UA |
| | | | | |

| ***product*** | ***Spectrum determination*** | | | |
|---|---|---|---|---|
| | ***Fresh*** | ***12 months*** | ***24 months*** | |
| L-Proline | 1.01 mg/ml | 1,00 mg/ml | 1,00 mg/ml | |

## Claims

1. Use of L-proline for manufacturing a pharmaceutical composition for the treatment of vitiligo.

2. Use according to claim 1, wherein the pharmaceutical composition contains L-proline in an alcoholic vehicle.

3. Use according to claim 2, wherein the pharmaceutical composition contains 0.5-2 mg/ml of L-proline in an alcoholic vehicle.

4. Use according to claim 2 or claim 3, wherein the alcoholic vehicle is 70° to 90° ethanol.

5. Use according to any one of claims 1-4, wherein the pharmaceutical composition is a composition for topical application.

6. Pharmaceutical composition for treatment of vitiligo, comprising L-proline as active principle in an alcoholic vehicle of 70° to 90° ethanol.

7. Pharmaceutical composition according to claim 6, which contains 0.5-2 mg/ml of L-proline.

8. Pharmaceutical composition according to claim 6 or claim 7, wherein the pharmaceutical composition is a composition for topical application.

## Patentansprüche

1. Verwendung von L-Prolin zur Herstellung einer pharmazeutischen Zusammensetzung für die Behandlung von Vitiligo.

2. Verwendung nach Anspruch 1, wobei die pharmazeutische Zusammensetzung L-Prolin in einem alkoholischen Vehikel enthält.

3. Verwendung nach Anspruch 2, wobei die pharmazeutische Zusammensetzung 0,5 - 2 mg/ml L-Prolin in einem alkoholischen Vehikel enthält.

4. Verwendung nach Anspruch 2 oder Anspruch 3, wobei das alkoholische Vehikel 70° bis 90° Ethanol ist.

5. Verwendung nach einem der Ansprüche 1 - 4, wobei die pharmazeutische Zusammensetzung eine Zusammensetzung für topische Anwendung ist.

6. Pharmazeutische Zusammensetzung für Behandlung von Vitiligo, die L-Prolin als aktiven Bestandteil in einem alkoholischen Vehikel aus 70° bis 90° Ethanol enthält.

7. Pharmazeutische Zusammensetzung nach Anspruch 6, die 0,5 - 2 mg/ml L-Prolin enthält.

8. Pharmazeutische Zusammensetzung nach Anspruch 6 oder Anspruch 7, wobei die pharmazeutische Zusammensetzung eine Zusammensetzung für topische Anwendung ist.

## Revendications

1. Utilisation de L-proline pour la fabrication d'une composition pharmaceutique destinée au traitement du vitiligo.

2. Utilisation selon la revendication 1, où la composition pharmaceutique contient de la L-proline dans un véhicule alcoolique.

3. Utilisation selon la revendication 2, où la composition pharmaceutique contient 0,5 à 2 mg/ml de L-proline dans un véhicule alcoolique.

4. Utilisation selon la revendication 2 ou la revendication 3, où le véhicule alcoolique est de l'éthanol à 70 à 90°.

5. Utilisation selon l'une quelconque des revendications 1 à 4, où la composition pharmaceutique est une composition destinée à l'application topique.

6. Composition pharmaceutique pour le traitement du vitiligo, comprenant comme principe actif de la L-proline dans un véhicule alcoolique constitué d'éthanol à 70 à 90°.

7. Composition pharmaceutique selon la revendication 6, qui contient 0,5 à 2 mg/ml de L-proline.

8. Composition pharmaceutique selon la revendication 6 ou la revendication 7, la composition pharmaceutique étant une composition destinée à l'application topique.
